Europäisches Patentamt

(19) European Patent Office

Office européen des brevets

(11) Publication number : **0 050 154**

**B1**

(12) **EUROPEAN PATENT SPECIFICATION**

(45) Date of publication of patent specification :
23.05.84

(51) Int. Cl.³ : **A 61 K 39/23**

(21) Application number : **81901246.9**

(22) Date of filing : **17.04.81**

(86) International application number :
**PCT/US 81/00508**

(87) International publication number :
**WO WO/81029 (29.10.81 Gazettee 81/25)**

(54) **MODIFIED LIVING CANINE PARVOVIRUS VACCINE.**

(30) Priority : **18.04.80 US 141447**

(43) Date of publication of application :
**28.04.82 Bulletin 82/17**

(45) Publication of the grant of the patent :
**23.05.84 Bulletin 84/21**

(84) Designated contracting states :
**CH DE FR GB LI NL SE**

(73) Proprietor : **CORNELL RESEARCH FOUNDATION, INC.**
**Day Hall Cornell University**
**Ithaca, NY 14853 (US)**

(72) Inventor : **CARMICHAEL, Leland E.**
**122 Pine Tree Road**
**Ithaca, NY 14850 (US)**
Inventor : **APPEL, Max J.G.**
**13 Redwood Lane**
**Ithaca, NY 14850 (US)**
Inventor : **McGREGOR, Douglas D.**
**160 Snyder Hill Road**
**Ithaca, NY 14850 (US)**

(74) Representative : **Kraus, Walter, Dr. et al**
**Patentanwälte Kraus & Weisert Irmgardstrasse 15**
**D-8000 München 71 (DE)**

(56) References cited :
US-A- 2 136 131
US-A- 2 271 818
US-A- 2 271 819
US-A- 3 133 861
US-A- 3 285 817
US-A- 3 293 130
US-A- 3 346 456
US-A- 3 465 077

(56) References cited :
US-A- 3 520 972
US-A- 3 562 387
US-A- 3 577 525
US-A- 3 709 782
US-A- 3 892 627
US-A- 3 944 469
US-A- 4 004 974
US-A- 4 193 990

US-A- 4 193 991
US-A- 4 211 843
US-A- 4 213 965

« A Research Update : Canine Parvovirus » Ralstom Purina Co. (1980)
Chappuis et al Le Point Vet 10 : 77-78 (1980)
Chapek et al Modern Vet. Pract. March 1980: 261-263
Appel et al VET. REC. 105: 156-159 (1979)
Hayes et al J.A.V.M.A. 174: 1197-1203 (1979)
Pollock et al MOD. VET. PRACTICE 60: 375-379 (1979)
Cooper et al CORNELL VET 69: 134144 (1979)
Appel et al CORNELL VET 69: 123-133 (1979)
Appel "CANINE PARVOVIRUS INFECTION" CORNELL RESEARCH LABORATORY FOR DISEASES OF DOGS, ALB. RPT. SER. 3 NO. 1 March 1979
Johnson et al AUTRALIAN VET. J. 55: 151 (1979)
Gagnon et al VET. RECORD 104: 263-264 (1979)
Black et al VET. MED/SMALL ANIM CLIN. January 1979: 47-50
Burtonboy et al ARCH. VIROL 61: 1-12 (1979)
Hayes et al CAN. VET. J. 20: 126 (1979)
Thomson et al CAN. VET. J. 19: 346 December 1978
Eugster et al J.A.V.M.A. 173: 1340-1341 November 15, 1978
Bachman et al INTERVIROLOGY 10: 1-16 (1978)
Appel et al J.A.V.M.A. 173: 1516-1518 (1978)
Eugster et al SOUTHWEST VET 30: 59-60 (1977)
Siegel « THE PARVOVIRUSES » in Gard et al VIROLOGY MONOGRAPHS 15 : 1 : 109 (1976) Springer/Verlag N.Y.
Johnson et al ARCHIV FOR DIE GESAMTE VIRUSFORSCHUNG 46 : 315-324 (1974)
Storz et al AM. J. VET. RES. 33: 269-272
Binn et al INFECTION AND IMMUNITY 1: 503-508 (1970)
Scott et al CORNELL VET. 60: 183-191 (1970)
Johnson J. SMALL ANIM. PRACTICE 8: 319-324 (1967)
Burger M.S. THESIS WASH STATE U. (1961) ABSTRACT « THE RELATIONSHIP OF MINK ENTERITIS VIRUS TO FELINE PANLEUCOPENIA VIRUS"
Gorham et al CORNELL VET 55: 554-566 (1965)
Burger et al SMALL ANIMAL CLINICAN November 1963: 611-614
BIOLOGICAL ABSTRACTS, vol. 71, 1981, page 6995, ref. 66689. & AM J VET RES 41(2): 2020-2024, 1980 A.K. FUGSTER: «Studies on canine parvovirus infections: Development of an Inactivated vaccine"
The file contains technical information submitted after the application was filed and not included in this specification

# 0 050 154

## Description

The field of the invention is that of virus vaccines for protection of dogs against infection by canine parvovirus, their production and use.

Parvoviruses are characterized as small animal DNA viruses consisting of an isometric protein capsid and a short molecule of single-stranded DNA. Although parvoviruses have been recovered and isolated from various animals, there had been no definite isolation of pathogenic canine parvovirus until recently (Siegl, The Parvoviruses, Springer-Verlag, New York 1976). Bachmann et al. include the dog as a possible parvovirus host in a report detailing the characteristics of parvoviruses in general (Bachmann et al., Intervirology 10 : in press, 1978). In 1970, Binn et al. reported the recovery and characterization of a « minute virus of canines » (Binn et al., Infect. Immun. 1 : 503, 1970). The isolates described were of canine origin, however, their pathogenicity was not known, and cytopathic effect (CPE) was produced in only a very narrow host range, i. e. only in a single continuous canine cell line, and not in primary canine nor primary or continuous cell cultures from other species. Pathogenicity for dogs was not determined nor was evaluation of vaccine potential done. Based on known properties of the Cornell isolates, it is clear that the recent CPV isolates are not the same as the « minute virus of canines » as described by Binn. In 1977, Eugster and Nairn reported a circumstantially-suggested causative link between diarrhea in puppies and a canine parvovirus. (Eugster, Nairn, Southwestern Veterinarian, 30 : 59, 1977). The isolate reported therein could not be serially propagated in MDCK cells, the only cell line tested. Again, pathogenic potential was unexamined and no experimental animal inoculations where performed. In 1978, widespread outbreaks of an apparently new disease in canines appeared (Appel, Cooper, Greisen and Carmichael, JAVMA 173(11) 1516-1518 ; Dec. 1978), occurring in both the United States and Australia (unpublished). The natural disease is characterized by diarrhea, fever, and leukopenia (relative lymphopenia).

The first isolation of a distinct parvovirus, and its in vitro propagation in primary cells and in cell lines of various species such as mink lung cells and canine kidney was described by two of the inventors in 105 Veterinary Record 156, and in their U. S. Patent N° 4,193,991, relating to a killed canine parvovirus vaccine.

The factors involved in selective genetic pressure on a virus were described in an article in 20 Infection and Immunity 108, April 1978, Carmichael & Medic, although that article dealt with a different virus (canine herpesvirus). A method of determining virulence through the use of plaque-size was disclosed in Inventor Carmichael's U.S. Patent N° 4,213,965, « Small Plaque Variant Canine Herpesvirus Vaccine ».

However, although heterotypic (Carmichael, et al, U.S. Patent N° 4,193,990) and killed vaccines (Carmichael, et al., Patent N° 4,193,991) have been known previously, until this invention there has been no successful production of a modified live vaccine for CPV. The production of a modified live CPV vaccine represents a novel and distinct advance in the art. A live virus vaccine constitutes a significant advance over heterotypic and killed vaccines because the magnitude of the immune response represents a 4- or 5-fold improvement over that produced by the other vaccines. The duration of immunity is vastly prolonged, and the live vaccine is commercially easier and cheaper to produce.

## Summary of the invention and description of preferred embodiment

The present invention relates to a method of protecting dogs against canine parvovirus. More specifically, the present invention relates to a method of producing a non-virulent attenuated living CPV variant from a native virulent strain of CPV for use as a safe, efficacious vaccine to protect dogs against infection caused by canine parvovirus. The attenuation of the virus is accomplished by prolonged serial passage in non-oncogenic (non-tumor-forming) cell cultures. Dogs vaccinated with the attenuated strain did not suffer illness when challenged by the native strain of CPV.

In the preferred embodiment of the invention, an attenuated live CPV vaccine is produced by serially passaging the native virulent virus (Cornell strain CPV-916) at least 108 times in mink lung and/or dog kidney cell lines at a sub-optimal (lower than that which is normal for the dog) temperature of 33 °C.

Other embodiments of the invention include use of other non-oncogenic cell cultures in addition to, or in place of, mink lung or dog kidney cells. An attenuated vaccine is produced with as few as 80 passages, in still another embodiment.

## Description of procedure and examples

Source of initial isolate :

The virulent native virus isolate used to begin the serial passaging was Cornell type strain 780916 (referred to as CPV-916). This strain (CPV-916) has been placed on deposit at The American Type Culture Collection (ATCC), Rockville, Maryland. The CPV-916 strain may be obtained from ATCC or The James A. Baker Institute for Animal Health, New York State College of Veterinary Medicine at Cornell University,

3

Ithaca, New York. The strain was recovered from the feces and intestinal contents of a Beagle pup that suffered acute diarrheal illness at the Argonne National Laboratories (September 9, 1978). Aggregates of the canine parvovirus (CPV) were initially detected by electron microscopy. Various non-oncogenic cell cultures were inoculated with this material.

CPV-916 was found to grow in a variety of cell types, as did additional CPV strains with indistinguishable properties. Cell cultures that supported growth of CPV were : (1) canine renal cells (primary, secondary, teriary), (2) feline renal cells (primary, Crandell FK cell line), (3) feline lung cell line (CCL 150), (4) Mink lung cell line (CCL 64), (5) MDCK cell line (CCL 34), (6) bovine fetal spleen cells, (7) bovine testicular cells.

The isolate selected for further passage was recovered in secondary dog renal cells prepared from the kidneys of a specific-pathogen-free (SPF) Beagle pup. This primary culture became « Cell Culture Passage # 1 ».

Serial propagation (« passage ») of CPV-916 and virulence tests :

1. Summary of technique : Primary or secondary canine renal cells from SPF dogs were chosen initially for serial passage of CPV-916. Initial passages at 3 to 4-day intervals (total of four) were made at 36°-37°C as undiluted virus. Each passage was monitored for presence of virus by increase in viral hemagglutinin activity (HA titer) and/or positive immunofluorescence (specific for CPV within cell nuclei). After 4 passages, dog inoculations indicated that the virus was virulent. Clinical signs included fever of variable duration (2 to 4 days), lymphopenia, decreased appetite, weight loss, depression, and loose, mucoid feces that sometimes contained blood. Virus at the 4th cell culture passage (referred to as CPV/4) was stored at − 70 °C. This « virulent virus » was later used for comparative pathogenicity studies and « challenge » of immunized dogs. Infectivity titrations of CPV/4 varied from $10^{6.5}$ to $10^{7.0}$ $TCID_{50}$ per 0.2 ml.

Subsequent passages of CPV (passage 5 and higher) were made at 3 to 5-day intervals using diluted (1 : 15-1 : 50) : virus. Incubation temperature initially (passages 1-31) was 36 °C ; subsequent passages were made at 33 °C. At various passage levels, the CPV was inoculated into one or more (usually two or three) Beagle dogs whose susceptibility had been previously determined by absence of hemagglutination-inhibiting (HI) antibody specific for CPV. In each trial, an equal number of littermate dogs was inoculated (« Challenged ») via oral, intramuscular, or intravenous routes with cell-culture-passaged CPV or CPV/4 (virulent), using similar viral doses and inoculation routes. Clinical and clinical-pathological signs were monitored closely each day and fecal viral shedding profiles were determined. Virus would be considered attenuated (modified in virulence by evidence of reduced pathogenicity) when the following conditions were satisfied : (1) absence of clinical signs in dogs inoculated with passaged virus and presence of signs in littermate control dogs inoculated with the reference (virulent) virus ; (2) reduction or absence of viral shedding in the feces of dogs given cell-culture passaged virus as compared with viral shedding profiles of dogs inoculated with reference virus.

2. CPV virulence at different passage levels (See Table 1 for summary) :

Passages-1 through-30 : The CPV was passaged at low (1 : 15-1 : 30) dilutions at 3 to 5-day intervals. No significant cytopathic effects were observed in primary or secondary canine renal cells at the time of passage, but viral growth was confirmed at each passage by the presence of intranuclear immunofluorescence, or assay in sensitive cell cultures.

Passage-30 (CPV/30) was tested for virulence by the intravenous inoculation of two 5-month-old SPF Beagles with $10^{6.0}$ $TCD_{50}$ of virus.

Both inoculated dogs had temperature increases (1.5 to 2 °C) on post-inoculation days (PID) 2 and 3. They had slight lymphopenia (PID 4-5), decreased appetites, and slight depression ; one dog had a loose, mucoid stool on PID 5. Both had hemagglutination-inhibition (HI) antibody levels of ≥ 5 120 by PID 14. It was thus determined that CPV/30 was still pathogenic for dogs.

Passages-31 through-51 : Additional passages in primary or secondary dog kidney cells continued, but at a reduced temperature (33 °C). This sub-optimal temperature was selected as it was the lowest at which satisfactory CPV growth occurred. Passage of other canine viruses at sub-optimal temperatures has resulted in the selection of variant strains of reduced pathogenicity (Carmichael and Medic, Infection and Immunity 20, 108, Apr. 1978 and U.S. Patent No 4,213,965, Carmichael Small Plaque Variant Canine Herpesvirus Vaccine). Passage-51 (CPV/51) was examined for virulence and immunogenicity for dogs.

In this trial, two dogs were used. One was inoculated by the subcutaneous + oral/nasal routes with 2 ml of CPV/51. The viral infectivity titer was $10^{6.2}$ per 0.2 ml ; the HA titer was 1 : 512. A sentinel (contact control) dog also was placed with the inoculated dog the following day to monitor viral shedding. Signs were monitored daily for 8 days.

The inoculated dog had slightly elevated temperatures PID 4 and 6, slight lymphopenia (PID 5 and 6) and a slightly mucoid, loose stool on PID 5. The contact control had a mild febrile response on PID 4 and a secondary febrile response on days 7 and 8. Lymphopenia was not detected in the contact animal. Viral shedding was evident from the serological response of the contact dog. By PID 18, both had antibody

4

titers in excess of 1 : 1,280.

Since SPF Beagle dogs have not responded to CPV with severe illness, any sign (slight temperature increase, lymphopenia, inappetence, depression, mucoid stool) was considered as unacceptable virulence for a potential vaccine strain. The slight temperature rise in the contact dog might be considered a pathological response to CPV, since subsequent seroconversion in this animal indicated viral spread from the inoculated dog. No other signs were observed in the contact animal.

Passages 52 through 79 : Continued viral passage was done at 33 °C in canine renal cells. Passages 64 and 73 were titrated and passaged at the endpoint dilutions ($10^{6.2}$ and $10^{6.5}$ $TCD_{50}$ per 0.2 ml, respectively). Other passages were done at 3 to 4-day intervals using diluted (1 : 50) virus. Cytopathic effects were not evident at the time of each harvest, but viral growth was determined by HA titrations at each passage (as noted above) and periodic infectivity assays by titration in cell cultures.

Passage 80 : An additional selective pressure on the virus population was introduced at this time. The 79th passage in dog renal cells (natural host cells) was inoculated into cell cultures derived from an alien host, i.e., mink lung cell line (American Type Culture Collection code CCL-64). This cell line (CCL-64) has been certified as free from mycoplasma, bacteria and fungi. It contains no known latent viruses. We have found it highly susceptible to CPV (Appel, Scott and Carmichael, Veterinary Record *105,* 156-159, 1979 and U. S. Patent N° 4,193,991, Carmichael and Appel, Canine Parvovirus Vaccine). All subsequent passages were performed in CCL-64 cells at 33 °C. CPV/80 was propagated at 33°-34 °C in CCL-64 cells in plastic flasks (75 cm) and harvested after 5 days growth. The infectivity titer was $10^{5.5}$ $TCD_{50}$/0.2 ml. Tests for virulence in dogs were then performed (Examples 1 and 2).

## Example 1

Two SPF Beagle dogs (855 and 854) were used : One animal (855) was inoculated intramuscularly with 1 ml of CPV/80 ; the second dog (854) was placed in contact the following day to monitor viral shedding. One dog (D855) was subsequently challenge-inoculated with CPV/4 to examine protective immunity.

The resulting data is summarized in Table II. The clinical response of D855 to CPV/80 was extremely mild ; the possibility of a slight lymphopenia on PID 5 suggested reduced virulence. There was no evidence of enteric disease, but CPV/80 virus was shed to the contact animal (D854). The contact dog developed HI antibody by PID 12, but remained clinically normal. Immunity challenge of D855 with virulent CPV/4, given intravenously 61 days after initial inoculation, indicated complete protection, and there was no shedding of the challenge virus from D855.

A clear reduction in natural virulence was apparent at passage 80, but the slight illness in SPF dogs indicated that this passage was less satisfactory than later (higher) passaged virus.

An additional experiment (Example 2) was therefore designed to further explore reduction in virulence.

## Example 2

Four littermate SPF Beagles were placed in barrier cages within isolation units and allocated to two groups. One group (dogs 862 and 863) was inoculated with $10^{6.5}$ $TCD_{50}$/0.2 ml (1 ml, intravenously) of CPV/80 ; dogs in the second group (dogs 863 and 864) each were inoculated by the same route with an identical dose of virulent CPV/4. Clinical responses were noted daily and fecal samples were collected for viral assay.

The resulting data is summarized in Table III. Salient findings were : (1) the clinical responses of the SPF dogs to both virulent and high-passaged (CPV/80) virus were mild, or indistinct. One dog given CPV/80 had a soft stool on FID 4, whilst normal stools were observed in all other animals at all post-inoculation times. Both dogs (864 and 865) given virulent virus had loss of appetite and were depressed on PID 3-4, but they were normal the following day. The dogs given CPV/80 were normal throughout the observation period.

Of greatest significance were the relative titers of virus found in the feces. Dogs given CPV/80 shed virus from PID 2-7, but the amount of virus shed was less than 1 % of that excreted by the dogs given virulent virus (shedding period : PID 2-6). When challenge-inoculated 6 months later, both dogs given CPV/80 were found immune.

Thus, the data suggested clear reduction in original virulence. The findings that CPV/80 was excreted later than CPV/4 and that amounts of excreted high-passaged virus were drastically reduced ($< 1 \%$) were especially significant. Reduced viral growth in a standard host may be taken as an indication of attenuation.

Passages 81 through 108 : Continued passage was done in CCL-64 cells at 3 to 4-day intervals. Passage 108 had an infectivity titer of $10^{7.5}$ $TCD_{50}$/0.2 ml. At that passage tests for virulence were done in outbred (not SPF) Beagle dogs obtained from a seronegative (susceptible) research kennel located in Ithaca, N. Y. Preliminary studies indicated that (unlike SPF dogs) animals from this kennel suffered more pronounced illness after inoculation with CPV/4 by the oral or intravenous routes. The dogs were used in Examples 3 and 4.

### Example 3

Six littermate dogs were divided into two isolated groups of 3 dogs each and then inoculated with CPV/108 or CPV/4. Two dogs in each group were given virus intramuscularly ; one dog in each group was oral-nasally inoculated. The viral doses (1 ml) were : CPV/108 = $10^{8.2}$ TCD$_{50}$ ; CPV/4 = $10^{7.7}$ TCD$_{50}$. Dogs were studied daily for clinical signs, viral shedding in the feces, and serological responses.

The resulting data is summarized in Table IV. The clinical-pathological responses of the dogs clearly discriminated between CPV/108 and virulent CPV/4. Animals given CPV/108 did not have clinical signs ; all gained weight and they developed high levels of HI antibody by PID 7. Viral shed in feces occurred with CPV/108, but amounts were less than 0.5 % of the quantity shed (time of maximal excretion) by the dogs given CPV/4. The period of viral shedding also was less. In contrast to the vaccinated dogs, all dogs given virulent CPV/4 became severely ill ; one dog (R-6) died on PID 10. Despite severe illness (inappetence, weight loss, severe depression on PIDs 6-9, and pasty, mucoid feces with blood), dogs R-4 and R-5 made rapid recoveries and were normal on PID 11. They remained gaunt for > 1 week. None had fever. Surprisingly, there was pronounced hypothermia in the most severely affected dogs.

This experiment indicated that attenuation of original virulence had occurred by passage 108. Characteristics of a suitable vaccine candidate strain seem satisfied at this passage level.

### Example 4

Immunogenicity of CPV/108 from dogs given $10^{5.2}$ TCD$_{50}$ by the intramuscular route was evaluated by challenge of immunity 28 days later. Serological and protective immune responses were evaluated. A sentinal animal (D890) was included to monitor viral shed in the feces. Results are recorded in Table V.

All three inoculated dogs remained healthy and developed high levels of HI antibody by PID 7. They were immune following challenge with virulent CPV/4. The contact animal remained normal but had HI antibody at PID 14. Virus shed from vaccinates to the contact dog, rendering it immune, though not directly injected.

### Example 5

Passage 115 (CPV/115) : Additional examination of virulence of high-passaged CPV was done at passage level 115 (CCL-64 cells ; 33 °C). Four outbred (non-SPF) 8-week-old Beagle pups were placed in separate isolation cages. Two pups (D870 and 871) were inoculated intravenously with 5 ml of CPV/115 ($10^{7.0}$ TCS$_{50}$/0.2 ml) ; two pups were given 5 ml of CPV/4 ($10^{6.5}$ TCD$_{50}$/0.2 ml) by the same route.

The two dogs given CPV/115 remained clinically normal throughout a 2-week observation period. In contrast, the pups given virulent virus both had mucoid, bloody feces and had febrile responses on PID 3-4. They were markedly depressed, unresponsive, and had inappetence on PID 3-6. There was only slight lymphopenia, but their erythrocyte sedimentation rates were increased, a clear sign of disease. Whereas dogs 870 and 871 (CPV/115-inoculated) gained approximately 1 1b during the first week after infection, dogs 872 and 873 (CPV/4-inoculated) lost 10 % of their initial body weights. Recovery was rapid, commencing on PID 6, but normal weights were not regained until 2 weeks later.

Pronounced differences in fecal shedding patterns also were found. Table VI illustrates comparative viral shedding patterns in the feces of dogs given CPV/4 or CPV/115. All dogs developed high HI antibody by PID 7.

When challenge-inoculated by the oral route as a test of immunity 3 weeks after vaccination, all dogs innoculated with CPV/115 were found immune. There was no illness, and virus was not shed in the feces.

Thus, it is shown that canine parvovirus at passage 115 (CPV/115) did not cause any signs of illness, but it engendered a protective immune response to virulent (CPV/4) virus.

The above CPV after 80 passages has been deposited with the American Type Culture Collection, Rockville, Maryland, as ATTCC VR 2017 ; and the above CPV after 115 passages has been deposited with the ATCC as VR 2016.

(See Table I page 7)

6

Table I.

Response of Beagle Dogs to Canine Parvovirus (Cornell Strain 916) at Various Cell Culture Passage Levels.

| Cell culture passage level | Inappetence (p.i.days) | Elevated temp. (p.i.days) | Lymphopenia (p.i.days) | Mucoid or bloody stool (p.i.days) |
|---|---|---|---|---|
| Passage-4* (35-36°C) | 7/7 (3-6) | 5/7 (3-4) | 3/5 (3-5) | 5/7 (4-10) |
| Passage-30 (35-36°C) | 2/2 (3-5) | 2/2 (4-6) | 2/2 (4-5) | 1/2 (5-6) |
| Passage-51 (33°C) | 0/1 | 1/1 (4) | 1/1 (4-5) | 0/1 |
| Passage-80 (33°C) | 0/2 | 0/2 | Not done | 0/2 |
| Passage-108 (33°C) | 0/3 | 0/3 | Not done | 0/3 |
| Passage-115 (33°C) | 0/2 | 0/2 | 0/2 | 0/2 |

Table I. (Continued)

Response of Beagle Dogs to Canine Parvovirus (Cornell Strain 916) at Various Cell Culture Passage Levels.

| Cell culture passage level | Weight loss | Death (p.i.day) | Viral Shed in Feces | Pathogenicity** | Remarks |
|---|---|---|---|---|---|
| Passage-4* (35-36°C) | 3/7 | 1/7 | Yes(3-10) | +++ | Virulent |
| Passage-30 (35-36°C) | Not done | 0/2 | Yes(3-7) | +++ | Virulent |
| Passage-51 (33°C) | 0/1 | 0/1 | Yes(4-7) | + or ± | Unacceptable virulence |
| Passage-80 (33°C) | 0/2 | 0/2 | Yes(3-7) | – | Reduced virulence |
| Passage-108 (33°C) | 0/3 | 0/3*** | Yes(3-7) | – | Avirulent |
| Passage-115 (33°C) | 0/2 | 0/2**** | | – | Avirulent |

*Various passage levels inoculated by intravenous route into S.P.F. Beagle pups less than 13 weeks of age. Viral dose varied from $10^{6.5}$ TCD$_{50}$/ml to $10^{7.5}$ TCID$_{50}$. In each experiment, at least 1 littermate control dog received virulent (CPV/4) virus.

**Pathogenicity score +−+++ refers to degree of illness or signs following infection.

***All (3) control littermates given CPV/4 became very sick, 1 died on dpi 10. Clear difference between CPV/3 and CPV/108 demonstrated.

****Control littermates (2 dogs) developed bloody, mucoid diarrhea p.i. days 3-4 ; had severe depression and weight loss. Clear demonstration of reduced pathogenicity of CPV/115 verus CPV/4.

7

Table II.

Test of Virulence (CPV/80) in Dogs

### D855C (inoculated)*

| Day post-inoc.(IM) | Temp. °C | Leuko-cytes (%lympho.) | Feces | H-I anti-body |
|---|---|---|---|---|
| -1 | 101.5 | 11.7(8.4) | Normal | < 10 |
| +1 | 101.2 | 11.2(8.2) | " | - |
| 2 | 101.8 | 16.9(6.7) | " | - |
| 3 | 101.5 | - | " | - |
| 4 | 101.5 | 10.9(5.5)** | " | 40 |
| 5 | 102.2 | 14.2(4.5) | " | 160 |
| 6 | 100.8 | 12.0(6.0) | " | 1280 |
| 7 | 101.2 | 14.2(7.1) | " | 2560 |
| ↓ | | | | |
| | 101.2. | --- | " | 2560 |

Immunity
↓ Challenge (IV) with virulent CPC***

| | | | | |
|---|---|---|---|---|
| 61 | 101.0 | --- | Normal | 5120 |
| 62 | 101.2 | --- | " | - |
| 63 | 101.2 | --- | " | - |
| 64 | 101.8 | 14.0(6.7) | " | - |
| 65 | 101.0 | --- | " | - |
| 66 | 101.2 | 17.1(7.2) | " | - |
| 67 | 101.8 | --- | " | 2560 |

Table II. (Continued)
Test of Virulence (CPV/80) in Dogs

### D854 (contact)

| Temp. °C | Leuko-cytes (%lympho.) | Feces | H-I anti-body |
|---|---|---|---|
| - | - | - | - |
| - | - | - | - |
| 101.5 | - | Normal | < 10 |
| 101.0 | - | " | |
| 101.7 | - | " | |
| 100.8 | - | " | |
| 101.5 | - | " | |
| 101.8 | - | " | < 10 |
| 101.8 | - | " | 640 |

8

Terminate

*Dog 855 inoculated intramuscularly (IM) with 1 ml (> $10^{5.5}$ TCD$_{50}$) of CPV/80. The following day D854 placed as in-unit contact to assess viral shedding from D855.

**Possible lymphopenia for this dog, but within normal range. Very slight temperature response on dpi 5.

***Immunity challenge 3 weeks post-vaccination with CPV/80. Dog given $\geq 10^{7}$ TCD$_{50}$ virulent CPV by intravenous (IV) route. No illness observed at any time.

Table III.

Disease and Fecal Shedding Patterns of CPV/80 Versus Virulent CPV/4

| Post-inoc. day | CPV/80 (I-V inoculation)* | | | | | | | |
| | Dog 862 | | | | Dog 863 | | | |
| | Temp. °F | Feces | Fecal CPV titer*** | HI anti-body | Temp. °F | Feces | Fecal CPV titer | HI anti-body |
| --- | --- | --- | --- | --- | --- | --- | --- | --- |
| 1 | 101.6 | Normal | <1.0 | <10 | 101.2 | Normal | <1.0 | <10 |
| 2 | 101.0 | " | <1.0 | – | 101.9 | " | <1.0 | – |
| 3 | 101.0 | " | 3.0 | <10 | 101.5 | " | 3.0 | <10 |
| 4 | 101.6 | Slightly soft | 5.2 | 160 | 101.6 | " | 4.5 | 40 |
| 5 | 101.3 | Normal | 6.5 | 320 | 101.0 | " | 5.8 | 640 |
| 6 | 101.6 | " | 5.2 | 1280 | 101.7 | " | 4.5 | >10,240 |
| 7 | 101.8 | " | <1.0 | 5120 | 101.4 | " | <1.0 | 10,240 |
| 8 | 101.8 | " | <1.0 | – | 101.2 | " | <1.0 | – |
| 9 | 101.1 | " | <1.0 | – | 101.0 | " | <1.0 | – |
| 10 | 101.8 | " | <1.0 | – | 101.0 | " | <1.0 | – |
| 11 | 102.0 | " | <1.0 | – | 101.0 | " | <1.0 | |
| 12 | – | " | – | – | – | " | – | – |
| 13 | – | " | – | – | – | " | – | – |
| 14 | – | " | – | 5120 | – | " | – | 5120 |
| ↓ | | | | | | | | |
| Oral Challenge with Virulent Virus | | | | | | | | |
| 180-187 | 101.5-101.8 | Normal | Neg. | 640 | 101.8-102.0 | Normal | Neg. | 640 |
| 188 | – | " | " | | – | " | | |

(See Tables III and IV page 10)

0 050 154

## Table III. (Continued)

### Disease and Fecal Shedding Patterns of CPV/80 Versus Virulent CPV/4

| | CPV/4 (I-V inoculation) | | | | | | | |
|---|---|---|---|---|---|---|---|---|
| | Dog 864** | | | | Dog 865 | | | |
| Post-inoc. day | Temp. °F | Feces | Fecal CPV titer | HI anti-body | Temp. °F | Feces | Fecal CPV titer | HI anti-body |
| 1 | 101.5 | Normal | <10 | <10 | 101.6 | Normal | <10 | <10 |
| 2 | 101.4 | " | 4.0 | – | 102.0 | " | 4.0 | – |
| 3 | 101.5 | " | $\bar{>}$ 9.0 | <10 | 102.2 | " | --- | <10 |
| 4 | 101.0 | " | 7.0 | 80 | 102.3 | " | $\bar{>}$ 9.0 | 80 |
| 5 | 102.2 | " | 6.5 | 1280 | 103.0 | " | 6.0 | 1280 |
| 6 | 101.2 | " | < 1.0 | >10,240 | 102.3 | " | < 1.0 | >10,240 |
| 7 | 101.6 | " | < 1.0 | 10,240 | 101.8 | " | < 1.0 | 10,240 |
| 8 | 101.2 | " | < 1.0 | – | 101.0 | " | < 1.0 | – |
| 9 | 101.2 | " | < 1.0 | – | 101.3 | " | < 1.0 | – |
| 10 | 101.8 | " | < 1.0 | – | 101.5 | " | < 1.0 | – |
| 11 | 101.0 | " | < 1.0 | – | 101.6 | " | < 1.0 | – |
| 12 | – | " | – | – | – | " | – | – |
| 13 | – | " | – | – | – | " | – | – |
| 14 | – | " | – | 5120 | – | " | – | 10,240 |

*SPF littermate Beagle pups inoculated intravenously (I-V) with either CPV/80 or virulent CPV (CPV/4). In both instances viral titers were $10^{6.5}/0.2$ ml $TCD_{50}$.

**Neither dog given virulent virus had clinical signs of enteritis, but both had anorexia, depression and were unresponsive p.i. days 3 and 4. None lost significant weight.

***Fecal CPV titer means $\log_{10}$ titer of infectious CPV per gm. feces, assayed in A/72 canine fibroblastic cells. Maximal viral titers are indicated by underline.

## Table IV.

### Disease Signs and Fecal Shedding Patterns of CPV/106 Versus Virulent CPV/4.

| | CPV Passage-108 | | | |
|---|---|---|---|---|
| | Dog R-1 (I-M)* | | | |
| Post-inoc. day | Temp. °F | Clinical illness | Fecal CPV titer (isol.) | HI anti-body |
| 1 | 101.6 | –** | < 20(-)*** | <10 |
| 2 | 100.6 | – | < 10(+) | --- |
| 3 | 100.4 | – | 40(+) | 10 |
| 4 | 101.0 | –. | --- | --- |
| 5 | 100.0 | – | 160(+) | 640 |
| 6 | 99.8 | – | --- | --- |
| 7 | 100.0 | – | 40(+) | 10,240 |
| 8 | 100.0 | – | --- | --- |
| 9 | 100.6 | – | --- | --- |
| 10 | 100.8 | – | $\bar{>}$ 20(-) | 10,240 |

10

Table IV. (Continued)

Disease Signs and Fecal Shedding Patterns of CPV/106V Versus Virulent CPV/4.

CPV Passage-108

Dog R-2 (I-M)

| Post-inoc. day | Temp. °F | Clinical illness | Fecal CPV titer (isol.) | HI anti-body |
|---|---|---|---|---|
| 1 | 101.0 | – | 10(–) | <10 |
| 2 | 100.7 | – | 20(+) | --- |
| 3 | 100.6 | – | 40(+) | 10 |
| 4 | 101.4 | – | --- | --- |
| 5 | 100.5 | – | 160(+) | 640 |
| 6 | 100.6 | – | --- | --- |
| 7 | 100.0 | – | 20(–) | 5120 |
| 8 | 101.4 | – | --- | --- |
| 9 | 100.4 | – | --- | --- |
| 10 | 99.4 | – | <10(–) | 5120 |

Table IV. (Continued)

Disease Signs and Fecal Shedding Patterns of CPV/106 Versus Virulent CPV/4.

CPV Passage 108

Dog R-3 (Oral/Nasal)

| Post-Inoc. day | Temp. °F | Clinical illness | Fecal CPV titer (isol.) | HI anti-body |
|---|---|---|---|---|
| 1 | 101.4 | – | 20(–) | <10 |
| 2 | 100.5 | – | 10(+) | --- |
| 3 | 100.7 | – | ≲10(+) | <10 |
| 4 | 100.9 | – | – | --- |
| 5 | 100.4 | – | 160(+) | 320 |
| 6 | 100.0 | – | – | --- |
| 7 | 100.1 | – | 10(+) | 10,240 |
| 8 | 100.6 | – | --- | --- |
| 9 | 100.5 | – | --- | --- |
| 10 | 100.3 | – | 10(–) | 10,240 |

Table IV. (Continued)

Disease Signs and Fecal Shedding Patterns of CPV/106 Versus Virulent CPV/4.

## Virulent CPV (Passage 4)

### Dog R-4 (I-M)

| Post-inoc. day | Temp. °F | Clinical illness | Fecal CPV titer (isol.) | HI anti-body |
|---|---|---|---|---|
| 1 | 101.0 | − | 10(−) | <10 |
| 2 | 101.0 | − | < 10(−) | <10 |
| 3 | 100.8 | − | 160(+) | <10 |
| 4 | 101.0 | − | --- | --- |
| 5 | 101.4 | − | > 20,480(+) | 640 |
| 6 | 100.8 | + | --- | --- |
| 7 | 100.4 | ++ | 320(+) | 10,240 |
| 8 | 99.0 | + | --- | --- |
| 9 | 100.2 | + | --- | --- |
| 10 | 100.8 | ± | 80(+) | 5120 |
| 11 | 100.0 | − | --- | --- |

Table IV. (Continued)

Disease Signs and Fecal Shedding Patterns of CPV/106 Versus Virulent CPV/4.

## Virulent CPV (Passage 4)

### Dog R-5 (I-M)

| Post-inoc. day | Temp. °F | Clinical illness | Fecal CPV titer (isol.) | HI anti-body |
|---|---|---|---|---|
| 1 | 101.0 | − | <10(−) | <10 |
| 2 | 100.8 | − | 40(−) | <10 |
| 3 | 102.0 | − | 2560(+) | <10 |
| 4 | 101.6 | − | --- | --- |
| 5 | 101.0 | + | >20,480(+) | 640 |
| 6 | 99.8 | ++ | --- | --- |
| 7 | 98.8 | +++ | 320(+) | 10,240 |
| 8 | 98.6 | ++ | --- | --- |
| 9 | 100.7 | ++ | --- | --- |
| 10 | 101.0 | ± | 80(−) | 5120 |
| 11 | 101.1 | − | --- | --- |

Table IV. (Continued)

Disease Signs and Fecal Shedding Patterns of CPV/106 Versus Virulent CPV/4.

Virulent CPV (Passage 4)

Dog R-6 (Oral/Nasal)

| Post-inoc. day | Temp, °F. | Clinical illness | Fecal CPV titer (isol.) | HI anti-body |
|---|---|---|---|---|
| 1 | 100.4 | − | 20(−) | <10 |
| 2 | 101.4 | − | 10(+) | --- |
| 3 | 101.1 | − | 160(+) | <10 |
| 4 | 100.0 | − | − | --- |
| 5 | 101.4 | + | >20,480(+) | 20 |
| 6 | 101.4 | ++ | − | |
| 7 | 101.1 | ++ | >10,240(+) | 20 |
| 8 | 99.2 | +++ | − | --- |
| 9 | 98.9 | +++ | − | --- |
| 10 | <95 | Died | (+) | --- |
| 11. | | | | |

*Dogs R-1, R-2, R-4, and R-5 inoculated by intramuscular (I-M) route, dogs R-3 and R-6 given virus by oral/nasal route. Viral titers : CPV/108 = $10^{7.5}$/0.2 ml ; CPV/4 = $10^{7.0}$/0.2 ml.

**Clinical illness was not observed (−) in R-1, R-2, or R-3 dogs at any time ; signs (+ to +++) in dogs R-4 and R-5 were pasty, mucoid feces with blood (dpi 5-10), depression, inappetence, and weight loss amounting to > 10 % body weight over a 6-day period, Dog R-6 died on p.i. day 10, with typical microscopic lesions of CPV.

***Fecal titers represent CPV hemagglutinin activity. Viral isolation indicated by (+ = pos.) or (− = neg.).

Table V.

Immune Response of Dogs to CPV/108 (Intramuscular Inoculation) and Viral Shed

| Dog | Status* | H-I Antibody Titer | | | | Response to challenge (PID28)** |
|---|---|---|---|---|---|---|
| | | Pre | 7 | 14 | 21 | |
| 880 | Vac. | <10 | 5120 | 2560 | 2560 | Immune |
| 881 | Vac. | <10 | 1280 | 2560 | 2560 | Immune |
| 882 | Vac. | <10 | 2560 | 2560 | 1280 | Immune |
| 883 | Contact | <10 | 10 | 1280 | 1280 | Immune |

*Dogs (880, 881, 882) vaccinated (Vac.) with $10^{5.2}$ $TCD_{50}$ of CPV/108 by intramuscular route. Dog 883 placed in contact with vaccinated to monitor viral shed.

**Dogs judged immune if there was no illness, seroconversion, or fecal viral shed following oral-nasal inoculation with $10^{6.5}$ $TCD_{50}$ of CPV/4 (virulent).

**0 050 154**

Table VI.

Response of Dogs to Canine Parvovirus Passage 115 (CPV/115) Versus Passage 4 (CPV/4).

Virus Inoculated

CVP/115* (Vaccinal Candidate)

| Post Inoc. Day | Dog 870 | | | | Dog 871 | | | |
|---|---|---|---|---|---|---|---|---|
| | Fecal Virus Titer** | | Serum H-I | Clin-ical | Fecal Virus Titer | | Serum H-I | Clin-ical |
| | HA | Infect. | Antibody | Signs | HA | Infect. | Antibody | Signs |
| 1 | <20 | <2 | <10 | -*** | <20 | <2 | <10 | - |
| 2 | <20 | <2 | --- | - | <20 | <2 | --- | - |
| 3 | <20 | >2 | --- | - | <20 | <2 | --- | - |
| 4 | 20 | 5.5 | --- | - | 20 | 4.5 | --- | - |
| 5 | <20 | 5.5 | --- | - | 80 | 3.5 | --- | - |
| 6 | <20 | 4.5 | --- | - | 640 | 3.5 | --- | - |
| 7 | 320 | 4.5 | 2560 | - | 640 | >2 | 1280 | - |
| 8 | 160 | 2 | --- | - | 80 | <2 | --- | - |
| 9 | <20 | <2 | --- | - | <20 | <2 | --- | - |
| ·10 | <20 | <2 | 5120 | - | <20 | <2 | 5120 | - |
| 14 | --- | --- | | | --- | --- | | |
| 21 | --- | --- | --- | | --- | --- | --- | - |
| Post-chall. Day | Challenge Inoculation (CPV/4 by oral-nasal route)† | | | | | | | |
| 1 | <20 | --- | 5120 | - | <20 | --- | 2560 | - |
| 2 | --- | | | - | --- | | | - |
| 3 | <20 | <2 | --- | - | <20 | <2 | --- | - |
| 4 | --- | | | - | --- | | | - |
| 5 | <20 | <2 | --- | - | <20 | <2 | --- | - |
| 6 | --- | | | - | --- | | | - |
| 7 | <20 | --- | 2560 | - | <20 | --- | 2560 | - |

(See Table VI page 15)

14

Table VI. (Continued)

Response of Dogs to Canine Parvovirus Passage 115 (CPV/115) Versus Passage 4 (CPV/4).

Virus Inoculated

CPV/4 (Virulent)

| Post Inoc. Day | Dog 872 | | | | Dog 873 | | | |
|---|---|---|---|---|---|---|---|---|
| | Fecal Virus Titer HA | Infect. | Serum H-I Antibody | Clinical Signs | Fecal Virus Titer HA | Infec. | Serum H-I Antibody | Clinical Signs |
| 1 | <20 | <2 | <10 | - | <20 | <2 | <10 | - |
| 2 | <20 | <2 | --- | - | <20 | <2 | --- | - |
| 3 | 320 | >2 | --- | + | 20 | 2 | --- | + |
| 4 | >327,680 | 8.2 | --- | +++ | >327,680 | 8.7 | --- | +++ |
| 5 | >327,680 | 9.7 | --- | +++ | >327,680 | 8.7 | --- | ++ |
| 6 | >327,680 | 9.0 | --- | + | >327,680 | 8.2 | --- | + |
| 7 | 10,240 | >2 | 5120 | + | <20 | >2 | 5120 | - |
| 8 | 320 | >2 | --- | - | <20 | <2 | --- | - |
| 9 | <20 | <2 | --- | - | <20 | <2 | --- | - |
| 10 | <20 | <2 | 5120 | - | <20 | <2 | 5120 | - |
| 14 | | | | | | | | |
| 21 | | | | | | | | |

*Dogs inoculated by intravenous route with either $10^{6.5}$ TCD$_{50}$ (CPV/4) or $10^{7.5}$ TCD$_{50}$ (CPV/115).
**Fecal virus titer expressed as hemagglutinating (HA) units or infectivity (Infect.)/gm feces.
***Clinical signs negative (−) or present (grades + to +++). Three-plus means fever, depression anorexia, mucoid and bloodtinged stool.
+ Only dogs given CPV/115 challenge-inoculated.

## Claims

1. A modified live vaccine for protecting dogs against infection caused by canine parvovirus produced by a process comprising the steps of taking a seed of a virulent canine parvovirus strain and serially passaging the strain in non-oncogenic cell cultures at temperatures lower than normal for the dog with periodic tests until a non-virulent strain is produced.

2. The vaccine of Claim 1, characterized by the fact that in the production process the serial passaging comprises at least 80 passages.

3. The vaccine of Claim 1, characterized by the fact that in the production process the passage temperature is approximately 33 °C.

4. The vaccine of Claim 1, characterized by the fact that in the production process the non-oncogenic cell culture consists of a medium selected from the group consisting of Maden-Darby Canine Kidney cell line (CCL-34), canine kidney cells, mink lung cells, bovine fetal spleen cells, bovine testicular cells, feline lung cells, or feline renal cells.

5. The vaccine of Claim 1, characterized by the fact that the virulent canine parvovirus strain is canine parvovirus strain 916.

6. A method of producing the modified live vaccine for protecting dogs against canine parvovirus according to Claims 1 to 5, characterized by the steps of
   a) Starting with a seed of virulent canine parvovirus ;
   b) Serially passaging the virus in non-oncogenic cell cultures at temperatures lower than normal for the dog ;

c) Periodically testing the passaged virus for virulence until a non-virulent virus is produced.

7. The method of Claim 6, in which the serial passaging comprises at least 80 passages.

8. The method of Claim 7, in which the serial passaging comprises 108 passages.

9. The method of Claim 6, in which the passage temperature is approximately 33 °C.

10. The method of Claim 6, in which the non-oncogenic cell culture consists of a medium selected from the group consisting of Maden-Darby Canine Kidney cell line (CCL-34), canine kidney cells, mink lung cells, bovine fetal spleen cells, bovine testicular cells, feline lung cells, or feline renal cells.

11. The method of Claim 6, in which the virulent canine parvovirus strain is canine parvovirus 916.

## Ansprüche

1. Modifizierter lebender Impfstoff zum Schutz von Wunden gegenüber einer durch Hunde-Parvovirus hervorgerufenen Infektion, dadurch gekennzeichnet, daß er durch ein Verfahren hergestellt worden ist, welches die Stufen umfaßt, daß man einen Samen eines virulenten Hunde-Parvovirusstammes nimmt und den Stamm der Reihe nach in nichtonkogenen Zellkulturen bei Temperaturen, die niedriger sind, als es für den Hund normal ist, unter periodischen Tests durchlaufen läßt, bis ein nichtvirulenter Stamm erzeugt wird.

2. Impfstoff nach Anspruch 1, dadurch gekennzeichnet, daß beim Herstellungsprozeß der der Reihe nach erfolgende Durchlauf mindestens 80 Durchläufe umfaßt.

3. Impfstoff nach Anspruch 1, dadurch gekennzeichnet, daß beim Herstellungsprozeß die Durchlauftemperatur ungefähr 33 °C ist.

4. Impfstoff nach Anspruch 1, dadurch gekennzeichnet, daß bei dem Herstellungsprozeß die nichtonkogene Zellkultur aus einem Medium, ausgewählt aus der Gruppe bestehend aus Maden-Darby Hundenierenzelllinie (CCL-34), Hundenierenzellen, Nerzlungenzellen, fetale Rindermilzzellen, Rinderhodenzellen, Katzenlungenzellen oder Katzennierenzellen, besteht.

5. Impfstoff nach Anspruch 1, dadurch gekennzeichnet, daß der virulente Hunde-Parvovirusstamm Hunde-Parvovirusstamm 916 ist.

6. Verfahren zur Herstellung des modifizierten lebenden Impfstoffs zum Schutz von Hunden gegen Hunde-Parvovirus nach den Ansprüchen 1 bis 5, gekennzeichnet durch die Stufen

a) Beginn mit einem Samen eines virulenten Hunde-Parvovirus,

b) der Reihe nach erfolgendem Durchlauf des Virus in nichtonkogenen Zellkulturen bei Temperaturen, die niedriger sind als es für den Hund normal ist,

c) periodisches Testen des durchgelaufenen Virus auf die Virulenz bis ein nichtvirulenter Virus erzeugt ist.

7. Verfahren nach Anspruch 6, dadurch gekennzeichnet, daß der der Reihe nach erfolgende Durchlauf mindestens 80 Durchläufe umfaßt.

8. Verfahren nach Anspruch 7, dadurch gekennzeichnet, daß der der Reihe nach erfolgende Durchlauf mindestens 108 Durchläufe umfaßt.

9. Verfahren nach Anspruch 6, dadurch gekennzeichnet, daß die Durchlauftemperatur ungefähr 33 °C ist.

10. Verfahren nach Anspruch 6, dadurch gekennzeichnet, daß die nichtonkogene Zellkultur aus einem Medium, ausgewählt aus der Gruppe bestehend aus Maden-Darby Hundenierenzelllinie (CCL-34), Hundenierenzellen, Nerzlungenzellen, fetale Rindermilzzellen, Rinderhodenzellen, Katzenlungenzellen oder Katzennierenzellen, besteht.

11. Verfahren nach Anspruch 6, dadurch gekennzeichnet, daß der virulente Hunde-Parvovirusstamm Hunde-Parvovirusstamm 916 ist.

## Revendications

1. Vaccin vivant modifié pour protéger des chiens contre l'infection provoquée par le parvovirus canin, caractérisé en ce qu'il est produit par un procédé selon lequel on prend un germe d'une souche de parvovirus canin virulente et qu'on fait passer en série la souche sur des cultures de cellules non oncogènes à des températures inférieures à une température normale pour le chien, en effectuant des tests périodiques jusqu'à ce qu'on produise une souche non virulente.

2. Vaccin selon la revendication 1, caractérisé en ce que dans le procédé de production, l'étape de passages en série comprend au moins 80 passages.

3. Vaccin selon la revendication 1, caractérisé en ce que dans le procédé de production, la température des passages est d'environ 33 °C.

4. Vaccin selon la revendication 1, caractérisé en ce que dans le procédé de production, la culture de cellules non oncogènes comprend un milieu choisi dans le groupe formé par une lignée de cellules de rein de chien Maden-Darby (CCL-34), des cellules de rein de chien, de cellules de poumons de vison, des cellules de rate fœtale de bovin, des cellules de testicules de bovin, des cellules de poumons de félin ou des cellules rénales de félin.

5. Vaccin selon la revendication 1, caractérisé en ce que la souche virulente de parvovirus canin est la souche 916 de parvovirus canin.

6. Procédé de production de vaccin vivant modifié pour protéger des chiens contre le parvovirus canin selon les revendications 1 à 5, caractérisé en ce que,

a) on démarre avec un germe de parvovirus canin virulent ;

b) on effectue des passages en série du virus sur des cultures de cellules non oncogènes, à des températures inférieures à une température normale pour le chien ;

c) on teste périodiquement le virus après passage pour déterminer sa virulence, jusqu'à ce qu'on ait produit un virus non virulent.

7. Procédé selon la revendication 6, caractérisé en ce que l'étape de passages en série comprend au moins 80 passages.

8. Procédé selon la revendication 7, caractérisé en ce que l'étape de passages en série comprend au moins 108 passages.

9. Procédé selon la revendication 6, caractérisé en ce que la température des passages est d'environ 33 °C.

10. Procédé selon la revendication 6, caractérisé en ce que la culture des cellules non oncogènes comprend un milieu choisi dans le groupe formé par une lignée de cellules de rein de chien Maden-Darby (CCL-34), des cellules de rein de chien, des cellules de poumons de vison, des cellules de rate fœtale de bovin, des cellules de testicules de bovin, des cellules de poumons de félin ou des cellules rénales de félin.

11. Procédé selon la revendication 6, caractérisé en ce que la souche virulente de parvovirus canin est la souche 916 de parvovirus canin.